# FASCICULE DE BREVET EUROPEEN

(11) **EP 2 445 476 B1**
(45) Date de publication et mention de la délivrance du brevet: **14.11.2018**
(21) Numéro de dépôt: 10745340.9
(22) Date de dépôt: 25.06.2010
(51) Int. Cl.: A61K 8/362, A61K 8/37, A61Q 17/04, A61K 8/97

(54) **COMPOSITION DESTINEE A LA PROTECTION SOLAIRE**
NAHRUNGSMITTEL FÜR DEN SONNESCHUTZ
FOOD COMPOSITION FOR SUN PROTECTION

(30) Priorité: 25.06.2009 FR 0954354
(43) Date de publication de la demande: 02.05.2012
(73) Titulaire: Biophytis, 75001 Paris (FR); SORBONNE UNIVERSITE, 75006 Paris (FR)
(72) Inventeur: VEILLET, Stanislas, F-91600 Savigny Sur Orge (FR); LAFONT René, F-75016 PARIS (FR); DIOH, Waly, F-91220 Bretigny Sur Orge (FR)
(74) Mandataire: Ipside
(86) Numéro de dépôt international: PCT/FR2010/051323
(87) Numéro de publication internationale: WO 2010/149942

(56) Documents cités:
- EP-A2- 1 484 051
- US-A- 5 032 382
- SACHAN K ET AL: "Bixa orellana L.- A dye yielding plant having medicinal properties" MEDICINAL & AROMATIC PLANTS ABSTRACTS, SCIENTIFIC PUBLISHERS, SCIENTIFIC PUBLISHERS, NEW DELHI - INDIA, vol. 25, no. 2, 1 avril 2003 (2003-04-01), XP018013015 ISSN: 0250-4367
- EVANS W C: "Annatto: a natural choice" BIOLOGIST, INSTITUTE OF BIOLOGY, LONDON, GB, vol. 47, no. 4, 1 septembre 2000 (2000-09-01), pages 181-184, XP009120450 ISSN: 0006-3347
- SRIVASTAVA A ET AL: "CHEMISTRY, PHARMACOLOGY AND USES OF BIXA ORELLANA - A REVIEW" JOURNAL OF MEDICINAL AND AROMATIC PLANT SCIENCES, UNKNOWN, IN, vol. 21, no. 21, 1 décembre 1999 (1999-12-01), pages 1145-1154, XP001070002
- AGNER A R ET AL: "DNA damage and aberrant crypt foci as putative biomarkers to evaluate the chemopreventive effect of annatto (Bixa orellana L.) in rat colon carcinogenesis" MUTATION RESEARCH. GENETIC TOXICOLOGY AND ENVIRONMENTALMUTAGENESIS, ELSEVIER, AMSTERDAM, NL, vol. 582, no. 1-2, 4 avril 2005 (2005-04-04), pages 146-154, XP004798787 ISSN: 1383-5718
- JONDIKO I J O ET AL: "Terpenoids and an apocarotenoid from seeds of Bixa orellana" PHYTOCHEMISTRY, PERGAMON PRESS, GB, vol. 28, no. 11, 1 janvier 1989 (1989-01-01), pages 3159-3162, XP026620780 ISSN: 0031-9422 [extrait le 1989-01-01]

## Description

### Domaine technique

L'invention concerne une composition destinée à un usage pour la protection de la peau contre les effets néfastes des rayonnements solaires ultraviolets.

### Etat de la technique

Il est connu que les rayonnements solaires peuvent être nocifs pour l'être humain. En effet, une grande partie de l'énergie solaire est émise sous forme de rayonnements UV, qui pénètrent profondément dans l'épiderme. La peau, pour se défendre de ces rayonnements, synthétise de la mélanine qui est un pigment apte à absorber les UV et à limiter leur pénétration profonde dans le derme. Cependant, le taux de mélanine synthétisée varie d'un individu à un autre et la filtration n'est pas suffisante pour protéger de manière sûre contre les agressions solaires. Notamment, les UVB de longueurs d'onde comprises entre 280 et 320 nm provoquent érythèmes et brûlures sur les peaux sensibles ou fréquemment exposées au soleil. De même, les UVA de longueurs d'onde comprises entre 320 et 400 nm, s'ils permettent le bronzage, sont également connus pour accélérer le vieillissement de la peau et sont impliqués dans le développement de nombreux cancers cutanés.

De nombreuses études ont été menées pour mettre au point des compositions, le plus souvent topiques, destinées à protéger la peau des agressions solaires lorsque celle-ci est exposée.

Ces compositions comportent fréquemment des caroténoïdes connus pour être utilisés par les plantes pour se protéger des agressions du soleil. Les caroténoïdes sont des pigments plutôt oranges et jaunes répandus chez de très nombreux organismes vivants. Ils appartiennent à la famille chimique des isoprénoïdes et sont liposolubles. Ils sont synthétisés par les algues, les plantes vertes et par de nombreux champignons et bactéries (dont les cyanobactéries). Les animaux ne sont pas capables de les produire, mais ils les trouvent dans leur nourriture.

Les caroténoïdes sont connus pour leur activité photoprotectrice, liée d'une part à leur absorption des rayons lumineux (rôle d'écran) et d'autre part à leurs propriétés antioxydantes, qui leur permettent de neutraliser efficacement les radicaux peroxyle et les molécules d'oxygène singulet (Sies et Stahl, 1995, 2004). Ils sont utlilisables par voie externe, mais également par voie interne, dans la mesure où une partie des caroténoïdes ingérés par les animaux se retrouve dans leur tégument. Des études de supplémentation avec le β-carotène et lycopène, seuls ou en mélange avec d'autres caroténoïdes montrent une photoprotection systémique, mesurée par une diminution des érythèmes induits par les UV.

Une étude clinique a montré qu'un mélange de caroténoïdes réduit le stress oxydatif. Cette étude clinique randomisée en double aveugle contre placebo décrit l'effet des caroténoïdes sur 32 individus non fumeurs et en bonne santé qui ont reçu quotidiennement et de façon croisée pendant des périodes de 3 semaines séparées par une pause de 12 semaines 4 capsules contenant chacune, soit 1 g d'huile de poisson, soit 1 g d'huile de poisson supplémentée avec un mélange de caroténoïdes (7,6 mg de caroténoïdes totaux dont 2,9 mg de bixine par capsule). Il a été observé que ce mélange de caroténoïdes réduit le stress oxydatif induit par la consommation de l'huile de poisson (très riche en acides gras polyinsaturés 20:5 et 20:6). Cela est illustré par une stabilité accrue des LDL dans un test d'oxydation *ex vivo* et la réduction de la concentration d'un marqueur de dégradation de l'ADN (la 8-hydroxy-2'-désoxyguanosine) au niveau des urines (Kiokias et Gordon, 2003).

Une autre étude chez la souris a montré que l'administration orale de divers caroténoïdes, dont la bixine (200 micromoles = 80 mg par kg de masse corporelle) avant une irradiation γ (1.5-3.0 Gy) réduit l'apparition de lésions chromosomiques, appréciée par le test des micronoyaux (Thresiamma *et al.,* 1998). La bixine, administrée per os préalablement à une chimiothérapie, est capable d'en réduire les effets secondaires (Silva et al., 2001).

L'activité antioxydante de la norbixine a aussi été étudiée sur de l'ADN plasmidique, en induisant des cassures radicalaires par le peroxyde d'hydrogène H₂O₂ en présence d'ions Sn²⁺ ou Fe²⁺ - réaction de Fenton : de faibles concentrations de norbixine (10 µM) protègent l'ADN mis en présence de 10 µM H₂O₂ (Kovary et al., 2001).

Par ailleurs, la norbixine augmente d'un facteur 10 la survie de bactéries (*E. coli*) soumises à des radiations UV, à du peroxyde d'hydrogène H₂O₂ ou des anions superoxyde O₂^{•-}. L'induction du système SOS par les UVC est réduite de 2,3 fois en présence de norbixine (Júnior *et al.,* 2005).

Enfin, la norbixine possède une activité antimutagène, et réduit ainsi de 87% l'effet mutagène de H₂O₂ sur *Salmonella typhimurium* (Kovary et al., 2001).

Plus globalement, il a été montré dans des systèmes acellulaires que les caroténoïdes peuvent neutraliser les anions superoxyde générés par le système xanthine / xanthine oxydase. Cette capacité de neutralisation dépend de la nature du caroténoïde et diminue dans l'ordre canthaxanthine > bixine > lutéine > β-carotène (Corol *et al.,* 2003). Pour ses effets antioxydants, il a ainsi été proposé d'ajouter de la bixine dans des aliments (Levy et Levy, 2003).

Sur cette base, des compositions contenant des caroténoïdes liposolubles, destinées à être appliquées sur la peau avant et au moment de l'exposition au soleil ont été développées afin de protéger celle-ci des méfaits du soleil. Leurs propriétés pigmentantes permettent de colorer simultanément les cellules de l'épiderme en orangé, accentuant l'impression de bronzage. Notamment, une composition cosmétique filtrante photostable a été développée pour la protection de l'épiderme humain contre les radiations UV, comprenant une quantité faible (0,0025-0,009 % en poids) de bixine dans un extrait huileux (Grollier et al., 1991).

Mais les caroténoïdes peuvent également être ingérés pour jouer leur rôle antioxydant au niveau notamment des cellules dermiques et épidermiques. Les composés ingérés sont en effet retrouvés dans le plasma puis dans divers tissus, dont la peau (Richelle et al., 2006). La peau humaine contient en moyenne de 0,2 à 0,6 ng/g de caroténoïdes totaux, avec de larges variations entre individus et entre zones du corps (Stahl et Sies, 2007). L'ingestion d'une nourriture supplémentée en caroténoïdes provoque une augmentation de la teneur du derme en caroténoïdes (Stahl *et al.,* 1998) et, pour être efficaces, ces derniers doivent être utilisés de façon prolongée, pendant une période supérieure à 10 semaines (Stahl *et al.,* 2000).

Une telle approche a montré son efficacité avec par exemple un mélange (30 à 90 mg/jour pendant 24 semaines) de β- et α-carotène (Lee *et al.,* 1999), avec un extrait de tomate apportant 16 mg/jour de lycopène pendant 12 semaines (Stahl *et al.,* 2001 ; 2006) ou un extrait d'algue *(Dunaniella salina)* apportant 24 mg/jour de β-carotène ou d'un mélange (1:1:1) de β-carotène, lutéine et lycopène pendant 12 semaines (Heinrich *et al.,* 2003). Les effets protecteurs ne se développent que progressivement, et l'administration d'une simple dose de 120 mg ou celle d'une dose quotidienne de 90 mg de β-carotène pendant 23 jours s'avèrent inefficaces (Garmyn et al., 1995). Toutefois, Kläui *et al.* (1973), qui proposent d'ingérer des mélanges de caroténoïdes contenant éventuellement de la bixine pour protéger la peau contre les UV, indiquent qu'un traitement commençant 10 à 20 jours avant l'exposition au soleil est suffisant.

Les caroténoïdes sont de façon générale des molécules pas ou peu toxiques (Agner *et al.,* 2004), même à des doses chroniques de 3 g/kg chez l'animal (Trumbo, 2005).

### Exposé de l'invention

L'urucum (*Bixa orellana*) appartient à la famille des bixacées. Cette plante se présente sous la forme d'un arbre de 5 à 10 mètres de hauteur, qui porte à l'extrémité de ses branches des fruits sous forme de cosses contenant des graines entourées d'une pulpe colorée en rouge orangé. L'urucum est utilisé pour la fabrication du colorant alimentaire, l'annatto (= E160b), qui est mélange de caroténoïdes contenant essentiellement de la bixine (C₂₅H₃₀O₄), un peu de norbixine (C₂₄H₂₈O₄), ainsi que plusieurs composés apparentés mineurs (Mercadante et al., 1997). La norbixine est un dérivé issu de l'hydrolyse alcaline de la bixine et est hydrosoluble. Diverses méthodes d'extraction du colorant à partir du péricarpe des graines d'urucum ont été décrites. Ces extraits sont dépourvus de toxicité (Hagiwara *et al.,* 2003 ; Bautista *et al.,* 2004). Les conclusions du JEFCA (Joint WHO/FAO Executive Committee on Food and Additives) tenu les 20-29 juin 2006 recommandent une dose journalière autorisée de 12 mg/kg de bixine pour un extrait titré à 92% de bixine et 0,6 mg/kg de norbixine pour un extrait titré à 91% de norbixine. Le E160b est utilisé par l'agroalimentaire pour colorer des matières grasses (beurre, huile, margarine), certains fromages, le riz, etc.

Les formules chimiques de la bixine et de la norbixine sont décrites ci-dessous.

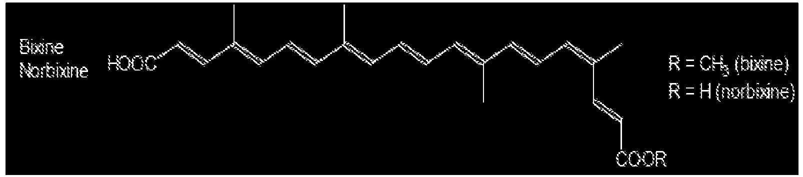

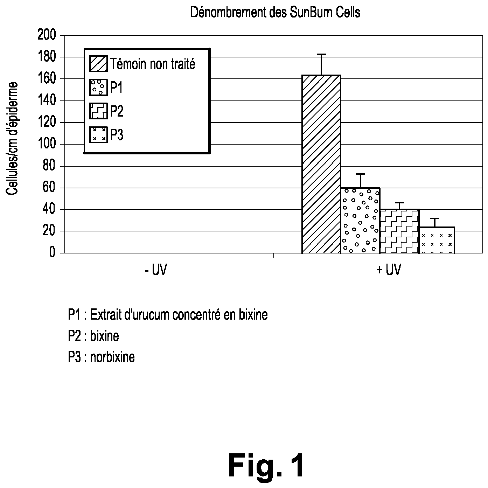

Les inventeurs ont découvert que la culture d'explants d'épiderme humain pendant une semaine en présence de bixine, norbixine ou extrait d'urucum (contenant ces deux molécules) entraîne une protection élevée à une irradiation UVB équivalent à environ 2 DEM.. Cette protection se traduit par la diminution très significative du nombre de cellules « coup de soleil » (sunburn cells ou SBC) observées 24 heures après l'irradiation (figure 1).

Les inventeurs ont observé que la diminution du nombre de SBC était plus importante sous l'effet de la norbixine (-84,4 %) que de la bixine (-74,4%) ou de l'extrait d'urucum (-63,9%). Cet effet de la norbixine est observé pour une concentration de 15,5 ng.mL⁻¹ (≈ 4.10⁻⁸ M).

Par ailleurs, une étude récente a montré un effet-dose de l'activité protectrice d'un extrait d'urucum enrichi en bixine à 166 ng.mL⁻¹ ; 330 ng.mL⁻¹ et 660 ng.mL⁻¹ vis à vis des altérations induites par une irradiation UV Cette étude montre une réduction respective de 15% à 166 ng.mL⁻¹, de 42% 330 ng.mL⁻¹ et de 63% à 660 ng.mL⁻¹.

Il est connu que la bixine ingérée est transformée en norbixine, qui est le principal composé retrouvé dans le plasma après ingestion de bixine. Suite à l'ingestion d'une solution huileuse d'annatto contenant 16 mg de bixine, des concentrations plasmatiques de norbixine supérieures à 25 ng.mL⁻¹ sont observées pendant plus de 6 heures, alors que celles de bixine n'atteignent que 10 ng.mL⁻¹, et ce pendant moins de 4 heures (Levy et al. 1997).

L'invention a donc pour objet une utilisation par voie orale d'une première molécule de formule (I) avec R=CH₃ pour prévenir les dommages causés par l'exposition aux rayons ultraviolets sur la peau des mammifères selon la revendication 1.

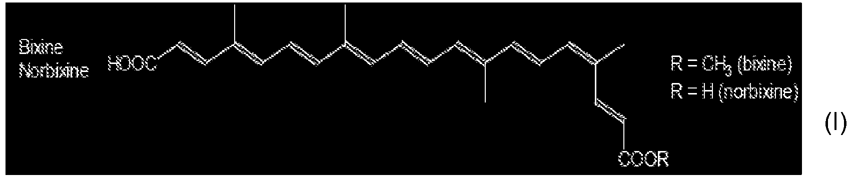

Dans un mode de réalisation de l'invention, la première molécule est mélangée à une deuxième molécule de formule (I) avec R = H.

Dans un mode de réalisation de l'invention, la première molécule est ingérée à une dose quotidienne comprise entre 5 mg et 100mg, de préférence à une dose quotidienne de 35mg.

Dans un exemple, la dose quotidienne peut être entre 30 et 40 mg pour une personne de 70 kg environ.

Dans un autre mode de réalisation de l'invention, la deuxième molécule est ingérée à une dose quotidienne comprise entre 5mg et 100mg, de préférence à une dose quotidienne de 35mg.

L'invention a également pour objet une composition destinée à être administrée par voie orale, pour la protection de la peau des mammifères contre les rayonnements UV, caractérisée en ce qu'elle comprend, dans un support acceptable pour être ingéré, une première molécule de formule (I) avec R= CH₃ à une dose minimale de 0,01% en poids de cette première molécule jusqu'à une dose maximale de 92% en poids de cette même première molécule.

Dans des exemples, la dose peut être de 0,001%, 0,01%, 0,1%, 1%, 2%, 5%, 7%, 10%, 15%, 20%, 25%, 30%, 35%, 40%, 45%, 50%, 55%, 60%, 65%, 70%, 75%, 80%, 85%, 90%.

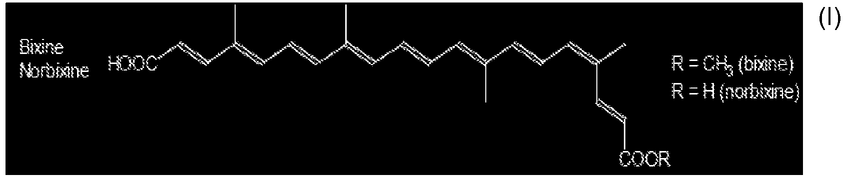

Dans un mode de réalisation de l'invention, la composition contient également une deuxième molécule de formule (I) avec R=H.

Dans un mode de réalisation de l'invention, la composition comprend une dose minimale de 0,001% en poids de la deuxième molécule jusqu'à une dose maximale de 92% en poids de cette même deuxième molécule. Dans des exemples, la dose peut être de 0,001%, 0,01%, 0,1%, 1%, 2%, 5%, 7%, 10%, 15%, 20%, 25%, 30%, 35%, 40%, 45%, 50%, 55%, 60%, 65%, 70%, 75%, 80%, 85%, 90%.

Dans un mode de réalisation de l'invention, selon la revendication 7, la première molécule est apportée sous forme d'un extrait d'urucum enrichi en la première molécule de formule (I) avec R = CH₃.

L'extrait d'urucum comporte entre 2 % et 92 % en poids de la première molécule, préférentiellement au moins 6% en poids de la première molécule

Dans un mode de réalisation de l'invention, la deuxième molécule est apportée sous forme d'extrait d'urucum enrichi la deuxième molécule de formule (I) avec R=H.

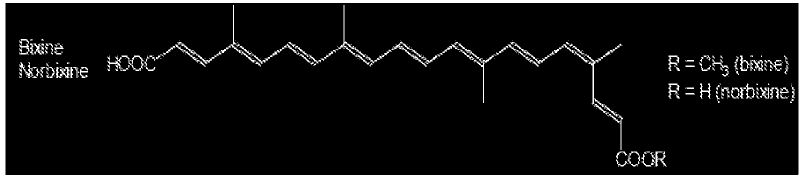

Dans un mode de réalisation de l'invention, l'extrait comporte entre 2 % et 92 % en poids de la deuxième molécule, préférentiellement au moins 6% en poids de la deuxième molécule.

Dans un mode de réalisation de l'invention, la composition est réalisée en tant que médicament ou complément alimentaire ou aliment.

L'invention a également pour objet un extrait d'urucum, caractérisé en ce qu'il est enrichi en une première molécule de formule (I) avec R= CH₃, lequel extrait contenant entre 2 % et 92% en poids, et préférentiellement au moins 6% en poids de la première molécule.

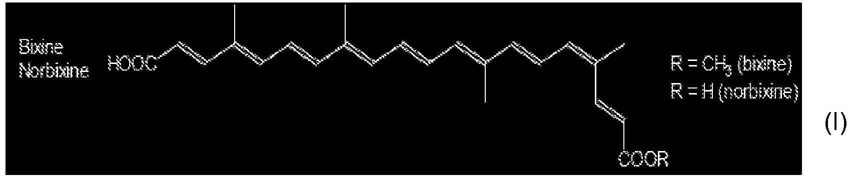

Dans un mode de réalisation de l'invention, l'extrait est également enrichi en une deuxième molécule de formule (I) avec R=H, lequel extrait contenant entre 2 % et 92% en poids, et préférentiellement au moins 6% en poids de la deuxième molécule.

Enfin, l'invention a pour objet l'extrait précédemment décrit pour formuler la composition précédemment décrite.

La première molécule de formule (I) avec R=H est appelée norbixine.

La deuxième molécule de formule (I) avec R=CH₃ est appelée bixine.

La quantité de bixine ou de norbixine recommandée par jour est de 35 mg (en 2 prises) pour obtenir un effet de protection contre les rayonnements solaires. Cette posologie est conseillée pour une personne pesant environ 70 Kg.

Le pourcentage inférieur en poids de la norbixine ou de la bixine est déterminé en fonction d'une concentration minimale en norbixine ou en bixine. La concentration minimale en norbixine ou en bixine est une concentration en norbixine ou en bixine qui en dessous de laquelle la norbixine ou la bixine selon l'invention n'a pas d'effet biologique ou effet photoprotecteur.

La norbixine ou la bixine peut être obtenue par extraction à partir de plantes contenant de la norbixine ou de la bixine. La plante qui est préférentiellement utilisée pour extraire la norbixine ou la bixine est l'urucum.

Par composition on entend produit alimentaire tel qu'une boisson, un produit laitier ou autre. La composition peut être une composition médicinale par exemple utilisée sous forme de pilules, de gélules ou de comprimés qui contiennent une dose bien précise de la première molécule de formule (I) avec R=H. Par composition médicinale, on entend une composition présentant des propriétés préventives vis-à-vis de rayonnements solaires sur la peau d'un mammifère.

La composition contient entre 2% et 92% en poids de la première molécule ou de la deuxième molécule et préférentiellement au moins 6% en poids de la première molécule ou de la deuxième molécule.

Par enrichi on entend que l'extrait comporte la première molécule ou la deuxième molécule en quantité telle qu'obtenue selon les procédés de préparation de l'extrait décrits ci-après.

La composition de gélules par exemple comprenant l'extrait enrichi est réalisée de sorte à ce qu'une gélule contienne 17,5 mg de bixine ou de norbixine. La fécule de manioc étant utilisée pour compléter la formule. Cela peut être par exemple 250 mg d'un extrait d'urucum à 7% de bixine et de 263 mg de fécule de manioc.

Le pourcentage en poids de la norbixine ou de la bixine présente dans l'extrait d'urucum est déterminé en fonction d'une concentration inférieure seuil en norbixine ou en bixine en-dessous de laquelle aucun effet (positif ou négatif) n'est observé. Le pourcentage en poids de la norbixine ou de la bixine présente dans cet extrait est aussi déterminé en fonction d'une concentration supérieure seuil au-delà de laquelle des effets néfastes pour la santé ou pour l'organisme ingérant cet extrait peuvent apparaître (inhibition de l'effet photoprotecteur, effet toxique, ....).

Comme précédemment mentionné, la composition s'entend par exemple d'un produit alimentaire tel qu'une boisson, un produit laitier ou autre. La composition peut être une composition médicinale par exemple utilisée sous forme de pilules, de gélules ou de comprimés qui contiennent une dose bien précise d'extrait enrichi en norbixine ou en bixine. Par composition médicinale, on entend une composition présentant des propriétés préventives vis-à-vis de rayonnements solaires sur la peau d'un mammifère.

La norbixine, la bixine, ou l'extrait d'urucum enrichi selon les revendications 1, 3, 7 et 9 peuvent être ingérés sous forme d'une gélule, d'un comprimé ou d'une pilule ou peuvent être mélangés à un produit alimentaire comme un fromage ou un yaourt ou un laitage.

Complément Alimentaire » a la signification donnée par la directive européenne 2002 /46/CE à savoir toute denrée alimentaire dont le but est de compléter le régime alimentaire normal et qui constitue une source concentrée de nutriments ou d'autres substances ayant un effet nutritionnel ou physiologique, seuls ou combines, commercialises sous forme de dose, a savoir, sous les formes de présentation telles que les gélules, les pastilles, les comprimes, le pilules et autres formes similaires ainsi que les sachets de poudre, les ampoules de liquide, les flacons munis d'un compte-gouttes et les autres formes analogues de préparation liquides ou en poudres destinées a être prises en unités mesurées de faible quantité.

« Aliment » a la signification donnée à l'article 2 de la réglementation européenne 178/2002 du Parlement européen. On entend par « aliment", toute substance ou produit, transformé, partiellement transformé ou non transformé, destiné à être ingéré ou raisonnablement susceptible d'être ingéré par l'être humain. Ce terme recouvre les boissons, les gommes à mâcher et toute substance, y compris l'eau, intégrée intentionnellement dans les denrées alimentaires au cours de leur fabrication, de leur préparation ou de leur traitement. Il inclut l'eau au point de conformité défini à l'article 6 de la directive 98/83/CE, sans préjudice des exigences des directives 80/778/CEE et 98/83/CE. Le terme "aliment" ne couvre pas:
- les aliments pour animaux;
- les animaux vivants à moins qu'ils ne soient préparés en vue de la consommation humaine;
- les plantes avant leur récolte;
- les médicaments au sens des directives 65/65/CEE(21) et 92/73/CEE du Conseil(22);
- les cosmétiques au sens de la directive 76/768/CEE du Conseil(23);
- le tabac et les produits du tabac au sens de la directive 89/622/CEE du Conseil(24);
- les stupéfiants et les substances psychotropes au sens de la Convention unique des Nations unies sur les stupéfiants de 1961 et de la Convention des Nations unies sur les substances psychotropes de 1971;
- les résidus et contaminants

« Médicament » a la signification donnée par la directive européenne 65/65/CE à savoir toute substance ou composition présentée comme possédant des propriétés curatives ou préventives à l'égard des maladies humaines ou animales et/ou toute substance ou composition pouvant être administrée à l'homme ou à l'animal en vue d'établir un diagnostic médical ou de restaurer, corriger ou modifier des fonctions organiques chez l'homme ou l'animal est également considérée comme médicament.

### Brève description de la figure

Figure 1 : Effets d'un extrait d'Urucum enrichi en bixine, de la bixine et de la norbixine sur le nombre de cellules « coup de soleil » suite à une irradiation de la peau par des UVB.

### 1) Exemple de procédé de préparation d'extrait d'urucum enrichi en bixine.

A 200 kg de graines d'urucum il est ajouté 400 litres d'éthanol absolu. Une première extraction est réalisée par agitation, pendant 16 heures. Une séparation par centrifugation est réalisée et 400 litres d'un premier extrait de liquide A est obtenu. Les résidus sont ré-extraits avec 400 litres d'éthanol absolu. Les deux extraits (800 litres) sont mélangés et ce volume est réduit à 80 litres ; on ajoute 10 kg de fécule de manioc et le tout est séché par atomisation : 30 kg d'un extrait sec sont ainsi obtenus.

### 2) Exemple de procédé de préparation d'un extrait d'urucum enrichi en norbixine.

La préparation de l'extrait enrichi en norbixine se fait par saponification de l'extrait riche en bixine par agitation dans une solution aqueuse de NaOH (bixine :NaOH 1 :3 mol/mol) pendant plusieurs heures à 37°C. Les échantillons de la réaction de saponification peuvent être analysés par HPLC jusqu'à une conversion bixine /norbixine proche de 100%. Le séchage se fait par évaporation de l'eau à 50°C et la Norbixine ainsi cristallisée pouvant se garder à -20°C. (Kovary *et al.,* 2001)

### 3) Etude expérimentale de l'effet d'un extrait d'Urucum enrichi en bixine, de la bixine et de la norbixine (figure 1).

Après traitement et irradiation UVB d'explants de peau humaine ex vivo, la coloration au trichrome de Masson permet la visualisation et le comptage des SBC.

### 3.1 Produits testés:

- P1 : Extrait d'urucum bio titré à 6 % en bixine (330 ng.mL⁻¹)
- P2 : Bixine pure (15,5 ng.mL⁻¹)
- P3 : Norbixine pure (15,5 ng.mL⁻¹)

### 3.2 Préparation des explants

27 explants d'un diamètre d'environ 10 mm ont été préparés à partir d'une plastie abdominale. Les explants ont été mis en survie en milieu de culture à 37°C en atmosphère humide, contenant 5 % de CO_{2.}

Ces explants ont été répartis en 9 lots comme suit :

| **Lot** | **Nb explants** | **Traitement** | **UV** | **Prélèvement** |
|---|---|---|---|---|
| Témoin J0 | 3 | Aucun | - | J0 |
| Témoin | 3 | Aucun | - | J8 |
| Extrait Urucum | 3 | Urucum | - | J8 |
| Bixine | 3 | Bixine | - | J8 |
| Norbixine | 3 | Norbixine | - | J8 |
| Témoin UV | 3 | Aucun | + | J8 |
| Extrait Urucum UV | 3 | Extrait Urucum UV | + | J8 |
| Bixine UV | 3 | Bixine UV | + | J8 |
| Norbixine UV | 3 | Norbixine UV | + | J8 |

### 3.3 Traitement

L'extrait d'urucum a été mis en solution dans du DMSO. Les trois produits ont été ajoutés dans le milieu de culture au premier jour (J0), de façon à atteindre les concentrations finales désirées. La quantité finale de DMSO dans le milieu est toujours restée inférieure à 1 %.

Les explants du lot T : témoin n'ont reçu aucun traitement.

### 3.4 Irradiation UV

Au jour J7, les explants ont été exposés aux UVB à la dose de 0,30 J/cm² et en UVA à la dose de 9 J/cm². Les lots non irradiés ont été placé à l'obscurité le temps des irradiations. Dès la fin de l'irradiation, les explants ont été remis en survie en milieu de culture.

### 3.5 Prélèvements

A J0, les 3 explants du lot Témoin T0 ont été prélevés et coupés en deux. Une partie a été fixée au Bouin ordinaire pour l'observation de la morphologie générale. L'autre a été congelée et conservée à -80°C.

A J8, soit 24 heures après l'irradiation, les 3 explants de chaque lot ont été prélevés et traités de la même façon.

### 3.6 Histologie

Après 48 heures de fixation dans le Bouin ordinaire, les prélèvements ont été déshydratés et imprégnés en paraffine à l'aide d'un automate de déshydratation Leica 1020. Les observations microscopiques ont été réalisées en microscopie optique, à l'aide d'un microscope Leica type DMLB, à l'objectif x 40.

### Examen de la morphologie générale et cellules coup de soleil

L'observation de la morphologie générale a été effectuée sur des coupes en paraffine après coloration au trichrome de Masson, variante de Goldner.

### Dénombrement des Sunburn Cells (SBC), figure 1

Les cellules coup de soleil (SBC) ont été dénombrées tout au long de chaque épiderme. La longueur de chaque épiderme a été mesurée à l'aide du module de mesure du logiciel Leica IM1000 et le nombre de SBC par cm d'épiderme a ensuite été calculé.

**Nombre de SBC par cm.**

| | Non irradié | | Irradié | |
|---|---|---|---|---|
| | Moyenne | Ecart type | Moyenne | Ecart type |
| Témoin non traité | 0,6 | 0,4 | 163,8 | 20,5 |
| P1 (Extrait d'Urucum) | 1,0 | 0,1 | 59,7 | 14,5 |
| P2 (Bixine) | 0,3 | 0,4 | 41,9 | 6,1 |
| P3 (Norbixine) | 0,3 | 0,4 | 25,5 | 8,7 |

Aucune SBC n'a été observée à T0.

Après 7 jours de traitement et exposition aux rayonnements ultraviolets, le produit P1, le produit P2 et le produit P3 entraînent respectivement une diminution significative de 63,9%, de 74,4% et de 84,4% du nombre de SBC dans l'épiderme par rapport à l'épiderme témoin non traité et irradié.

### Références bibliographiques

Programa conjunto FAO/OMS sobre normas alimentarias. Comision del codex alimentarius 29° periodo de sesiones Ginebra, suiza, 3-7 de julio de 2006.
Informe de la septima reunion del comité del codex sobre la leche y los productos lacteos queenstown, Nueva Zelandia 27 de marzo- 1° de abril de 2006. Summary and conclusions of the sixty-seventh meeting of the Joint FAO/WHO Expert committee on food Additives (JECFA) page 2 of 11 issued July 2006.
FDA Code of Federal Regulations. Title 21, Volume 1 Revised as of April 1, 2002. From the U.S. Government Printing Office via GPO Access [CITE: 21CFR73.30] Sec. 73.30 Annatto extract [Pages 346-347]
Agner A.R., Barbisan L.F., Scolastici C., Salvadori D.M. 2004. Absence of carcinogenic and anticarcinogenic effects of annatto in the rat liver medium-term assay. Food Chem. Toxicol. 42, 1687-1693.
Bautista A.R.P.L., Moreira E.L.T., Batista M.S., Miranda M.S., Gomes I.C.S. 2004. Subacute toxicity assessment of annatto in rat. Food Chem. Toxicol. 42, 625-629.
Biesalski H.K., Obermueller-Jevic U.C. 2001. UV light, beta-carotene and human skin - beneficial and potentially harmful effects. Arch. Biochem. Biophys. 389, 1-6.
Corol D-I., Dorobantu I.I., Toma N., Nitu R. 2003. Diversity of biological functions of carotenoids. Romanian Biotechnol. Lett. 8, 1067- 1074.
Fernandes A.C.S., Almeida C.A., Albano F., Laranja G.A.T., Felzenswalb I., Lage C.L.S., de Sa C.C.N.F., Moura A.S., Kovary K. 2002. Norbixin ingestion did not induce any detectable DNA breakage in liver and kidney but caused a considerable impairment in plasma glucose levels of rats and mice. J. Nutr. Biochem. 13, 411-420.
Garmyn M., Ribaya-Mercado J.D., Russell R.M., Bhawan J., Gilchrest B.A. 1995. Effect of β-carotene supplementation on human sunburn reaction. Exp. Dermatol. 4, 104-111.
Grollier J.F., Cotteret J., Rosenbaum G. 1991. Light-stable screening cosmetic composition containing bixin combined with a lipid-soluble UV filter and its use for protecting the human epidermis against ultra-violet radiation. Patent WO/5,032,382.
Hagiwara A., Imai N., Ichibara T., Sano M., Tamano S., Aoki H., Yasuhara K., Koda T., Nakamura M., Shirai T. 2003. A thirteen-week oral toxicity study of annatto extract (norbixin), a natural food color extracted from the seed coat of annatto (Bixa orellana L.), in Sprague-Dawley rats. Food Chem. Toxicol. 41, 1157 -1164.
Heinrich U., Gärtner C., Wiebusch M., Eichler O., Sies H., Tronnier H., Stahl W. 2003. Supplementation with β-carotene or a similar amount of mixed carotenoids protects Humans from UV-induced erythema. J. Nutr. 133, 98-101.
Júnior A.C.T.S., Asad L.M.B.O., de Oliveira E.B., Kovary K., Asad N.R., Felzenszwalb I. 2005. Antigenotoxic and antimutagenic potential of an annatto pigment (norbixin) against oxidative stress. Genet. Mol. Res. 4, 94-99.
Kiokias S. and Gordon M.H. 2003. Dietary supplementation with a natural carotenoid mixture decreases oxidative stress. Eur. J. Clin. Nutr. 57, 1135-1140.
Kläui H., Körner, W.F. 1973. Light-screening composition and method. US Patent 3,920,834.
Kovary K., Louvain T.S., Costa e Silva M.C., Albano F., Pires B.B.M., Laranja G.A.T., Lage C.L.S., Felzenszwalb I. 2001. Biochemical behaviour of norbixin during in vitro DNA damage induced by reactive oxygen species. Br. J. Nutr. 85, 431-440.
Levy L.W., Regalado E., Navarrete S., Watkins RH. 1997. Bixin and norbixin in human plasma: determination and study of the absorption of a single dose of Annatto food color. Analyst 122, 977-980.
Levy P.E., Levy L.W. 2003. Supplements containing annatto extracts and carotenoids and methods for using the same. Patent WO03/047528 A2.
Mercadante A.Z., Steck A., Pfander H. 1997. Isolation and structure elucidation of minor carotenoids from annatto (Bixa orellana L.) seeds. Phytochemistry 46, 1379-1383.
Richelle M., Sabatier M., Steiling H., Williamson G. 2006. Skin bioavailability of dietary vitamin E, carotenoids, polyphenols, vitamin C, zinc and selenium. Br. J. Nutr., 96, 227-238.
Russell K.R.M., Morrison E.Y.St.A., Ragoobirsingh D. 2005. The effect of annatto on insulin binding properties in the dog. Phytother. Res. 19, 433-436.
Sies H., Stahl W. 1995. Vitamins E and C, β carotene and other carotenoids as antioxydants. Am. J. Clin. Nutr., 62, 1315S-1321S.
Sies H., Stahl W. 2004. Carotenoids and UV protection. Photochem. Photobiol. Sci., 3, 749-752.
Silva C.R., Antunes L.M.G., Bianchi M.D.L.P. 2001. Antioxidant action of bixin against cisplatin-induced chromosome aberrations and lipid peroxidation in rats. Pharmacol. Res. 43, 561-566.
Stahl W., Heinrich U., Jungmann H., von Laar J., Schietel M., Sies H., Tronnier H. 1998. Increased dermal carotenoid levels assessed by noninvasive reflection spectrophotometry correlate with serum levels in women ingesting betatene. J. Nutr. 128, 903-907.
Stahl W., Heinrich U., Jungmann H., Sies H., Tronnier H. 2000. Carotenoids and carotenoids plus vitamin E protect against ultraviolet light-induced erythema in humans. Am. J. Clin. Nutr. 71, 795-798.
Stahl W., Heinrich U., Wiseman S., Eichler O., Sies H., Tronnier H. 2001. Dietary tomato paste protects against ultraviolet light-induced erythema in humans. J. Nutr. 131, 1449-1451.
Stahl W., Heinrich U., Aust, O., Tronnier H., Sies H. 2006. Lycopene-rich products and dietary photoprotection. Photochem. Photobiol. Sci. 5, 238-242.
Stahl W., Sies H. 2007. Carotenoids and flavonoids contribute to nutritional protection against skin damage from sunlight. Mol. Biotechnol. 37, 26-30.
Thresiamma K.C., George J., Kuttan R. 1998. Protective effect of curcumin, ellagic acid and bixin on radiation induced genotoxicity. J. Exp. Clin. Cancer Res., 4, 431-43.
Trumbo P.R. 2005. Are there adverse effects of lycopene exposure ? J. Nutr. 135, 2060S-2061S.
Young A.J., Lowe G.M. 2001. Antioxidant and prooxidant properties of carotenoids. Arch. Biochem. Biophys. 385, 20-27.

## Revendications

1. Première molécule de formule (I) avec R = CH₃ pour son utilisation pour prévenir les dommages causés par l'exposition aux rayons ultraviolets sur la peau des mammifères, **caractérisée** en qu'elle est administrée par voie orale.

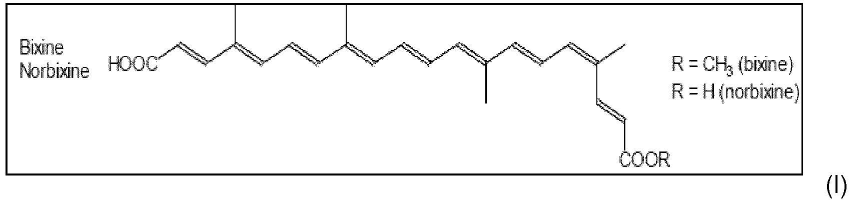

2. Première molécule de formule (I) avec R = CH₃ selon la revendication 1, pour son utilisation par voie orale pour prévenir les dommages causés par l'exposition aux rayons ultraviolets sur la peau des mammifères, **caractérisée en ce que** ladite première molécule est mélangée à une deuxième molécule de formule (I) avec R = H.

3. Première molécule de formule (I) avec R = H pour son utilisation pour prévenir les dommages causés par l'exposition aux rayons ultraviolets sur la peau des mammifères, **caractérisée** en qu'elle est administrée par voie orale.

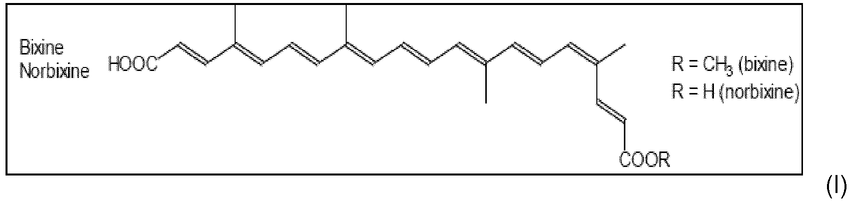

4. Première molécule de formule (I) avec R = H selon la revendication 3, pour son utilisation par voie orale pour prévenir les dommages causés par l'exposition aux rayons ultraviolets sur la peau des mammifères, **caractérisée en ce que** ladite première molécule est mélangée à une deuxième molécule de formule (I) avec R = CH₃.

5. Première molécule de formule (I) selon l'une des revendications 1 à 2 ou selon l'une des revendications 3 à 4, pour son utilisation par voie orale pour prévenir les dommages causés par l'exposition aux rayons ultraviolets sur la peau des mammifères, par ingestion d'une dose quotidienne de ladite première molécule comprise entre 5 mg et 100 mg, de préférence une dose quotidienne de 35 mg.

6. Première molécule de formule (I) selon la revendication 2 ou selon la revendication 4, pour son utilisation par voie orale pour prévenir les dommages causés par l'exposition aux rayons ultraviolets sur la peau des mammifères, par ingestion d'une dose quotidienne de ladite deuxième molécule comprise entre 5 mg et 100 mg, de préférence à une dose quotidienne de 35 mg.

7. Extrait d'Urucum comportant entre 2 % et 92 % en poids d'une première molécule de formule (I) avec R = CH₃ pour son utilisation pour prévenir des dommages causés par l'exposition aux rayons ultraviolets sur la peau des mammifères, **caractérisée** en qu'elle est administrée par voie orale.

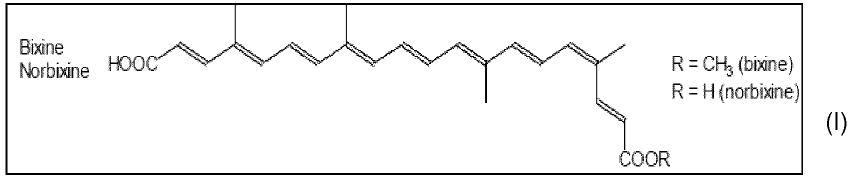

8. Extrait d'Urucum selon la revendication 7, ledit extrait comportant également entre 2 % et 92 % en poids d'une deuxième molécule de formule (I) avec R = H.

9. Extrait d'Urucum comportant entre 2 % et 92 % en poids d'une première molécule de formule (I) avec R = H pour son utilisation pour prévenir des dommages causés par l'exposition aux rayons ultraviolets sur la peau des mammifères, **caractérisée** en qu'elle est administrée par voie orale.

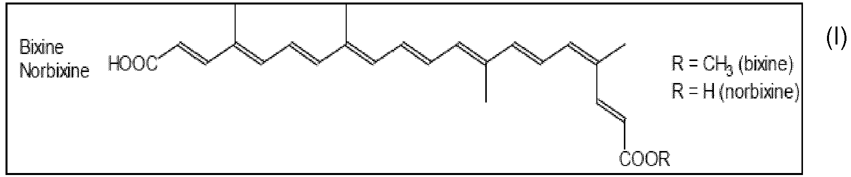

10. Extrait d'Urucum selon la revendication 9, ledit extrait comportant également entre 2 % et 92 % en poids d'une deuxième molécule de formule (I) avec R = CH₃.

11. Extrait d'Urucum selon l'une des revendications 7 à 8 ou selon l'une des revendications 9 à 10, ledit extrait comportant au moins 6% en poids de la première molécule.

12. Extrait d'Urucum selon la revendication 8 ou selon la revendication 10, ledit extrait comportant au moins 6% en poids de la deuxième molécule.

## Patentansprüche

1. Erstes Molekül der Formel (I) mit R = CH₃ für seine Verwendung zur Prävention der Schäden, die vom Aussetzen an ultraviolette Strahlen auf der Haut der Säugetiere verursacht werden, **dadurch gekennzeichnet, dass** es auf oralem Weg verabreicht wird.

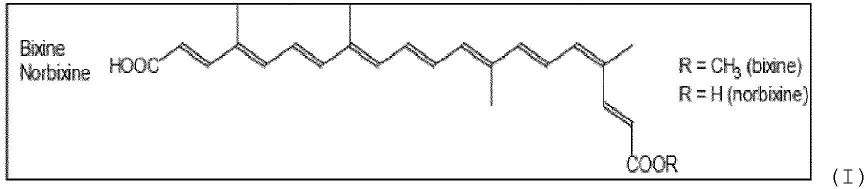

2. Erstes Molekül der Formel (I) mit R = CH₃ nach Anspruch 1 für seine Verwendung auf oralem Weg, zur Prävention der Schäden, die vom Aussetzen an ultraviolette Strahlen auf der Haut der Säugetiere verursacht werden, **dadurch gekennzeichnet, dass** das erste Molekül mit einem zweiten Molekül der Formel (I) mit R = H gemischt wird.

3. Erstes Molekül der Formel (I) mit R = H für seine Verwendung zur Prävention der Schäden, die vom Aussetzen an ultraviolette Strahlen auf der Haut der Säugetiere verursacht werden, **dadurch gekennzeichnet, dass** es auf oralem Weg verabreicht wird.

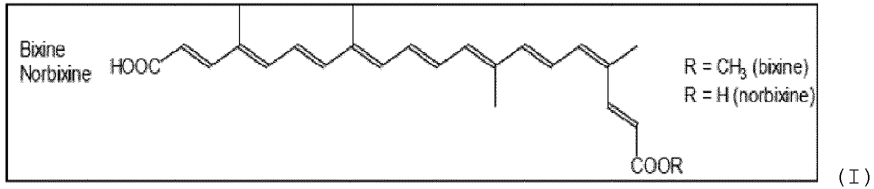

4. Erstes Molekül der Formel (I) mit R = H nach Anspruch 3, für seine Verwendung auf oralem Weg, um den Schäden vorzubeugen, die vom Aussetzen an ultraviolette Strahlen auf der Haut der Säugetiere verursacht werden, **dadurch gekennzeichnet, dass** das erste Molekül mit einem zweiten Molekül der Formel (I) mit R = CH₃ gemischt wird.

5. Erstes Molekül der Formel (I) nach einem der Ansprüche 1 bis 2 oder nach einem der Ansprüche 3 bis 4, für seine Verwendung auf oralem Weg, zur Prävention der Schäden, die vom Aussetzen an ultraviolette Strahlen auf der Haut der Säugetiere verursacht werden, durch Einnahme einer täglichen Dosis des ersten Moleküls, die zwischen 5 mg und 100 mg liegt, vorzugsweise einer täglichen Dosis von 35 mg.

6. Erstes Molekül der Formel (I) nach Anspruch 2 oder nach Anspruch 4 für seine Verwendung auf oralem Weg, um den Schäden vorzubeugen, die vom Aussetzen an ultraviolette Strahlen auf der Haut der Säugetiere verursacht werden, durch Einnahme einer täglichen Dosis des zweiten Moleküls, die zwischen 5 mg und 100 mg liegt, vorzugsweise mit einer täglichen Dosis von 35 mg.

7. Urucum-Extrakt, umfassend zwischen 2 und 92 Gew% eines ersten Moleküls der Formel (I) mit R = CH₃ für seine Verwendung zur Prävention der Schäden, die vom Aussetzen an ultraviolette Strahlen auf der Haut der Säugetiere verursacht werden, **dadurch gekennzeichnet, dass** es auf oralem Weg verabreicht wird.

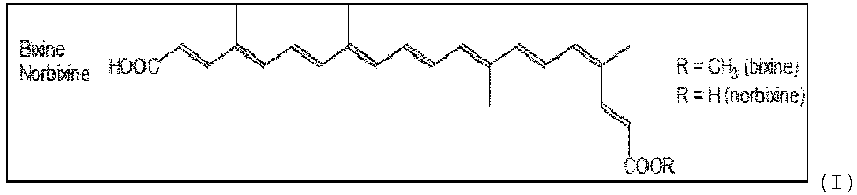

8. Urucum-Extrakt nach Anspruch 7, wobei der Extrakt ebenfalls zwischen 2 und 92 Gew% eines zweiten Moleküls der Formel (I) mit R = H umfasst.

9. Urucum-Extrakt, umfassend zwischen 2 und 92 Gew% eines ersten Moleküls der Formel (I) mit R = H für seine Verwendung zur Prävention der Schäden, die vom Aussetzen an ultraviolette Strahlen auf der Haut der verursacht werden, **dadurch gekennzeichnet, dass** es auf oralem Weg verabreicht wird.

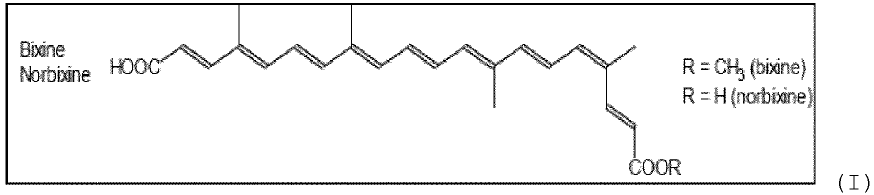

10. Urucum-Extrakt nach Anspruch 9, wobei das Extrakt ebenfalls zwischen 2 und 92 Gew% eines zweiten Moleküls der Formel (I) mit R = CH₃ umfasst.

11. Urucum-Extrakt nach einem der Ansprüche 7 bis 8 oder nach einem der Ansprüche 9 bis 10, wobei der Extrakt mindestens 6 Gew% des ersten Moleküls umfasst.

12. Urucum-Extrakt nach Anspruch 8 oder nach Anspruch 10, wobei der Extrakt mindestens 6 Gew% des zweiten Moleküls umfasst.

## Claims

1. First molecule having formula (I) with R = CH₃ for its use in preventing the damage caused by exposure to ultraviolet rays on the skin of mammals, **characterised in that** it is administered orally.

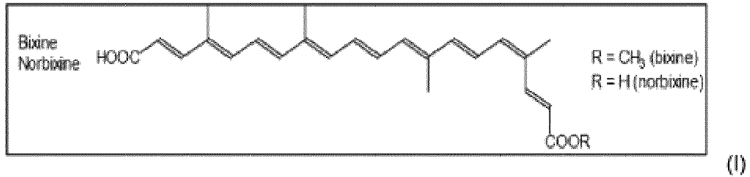

2. First molecule having formula (I) with R = CH₃ according to claim 1, for its use orally for preventing the damage caused by exposure to ultraviolet rays on the skin of mammals, **characterised in that** said first molecule is mixed with a second molecule having formula (I) with R = H.

3. First molecule having formula (I) with R = H for its use in preventing the damage caused by exposure to ultraviolet rays on the skin of mammals, **characterised in that** it is administered orally.

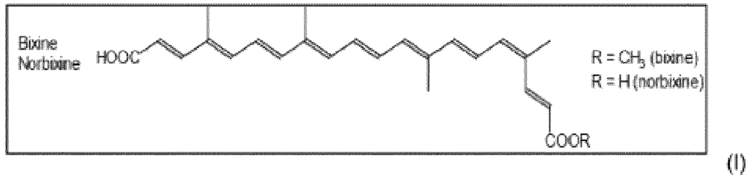

4. First molecule having formula (I) with R = H according to claim 3, for its use orally for preventing the damage caused by exposure to ultraviolet rays on the skin of mammals, **characterised in that** said first molecule is mixed with a second molecule having formula (I) with R = CH₃.

5. First molecule having formula (I) according to one of claims 1 to 2 or according to one of claims 3 to 4, for its use orally for preventing the damage caused by exposure to ultraviolet rays on the skin of mammals, by ingestion of a daily dose of said first molecule between 5 mg and 100 mg, preferably a daily dose of 35 mg.

6. First molecule having formula (I) according to claim 2 or according to claim 4, for its use orally for preventing the damage caused by exposure to ultraviolet rays on the skin of mammals, by ingestion of a daily dose of said second molecule between 5 mg and 100 mg, more preferably at a daily dose of 35 mg.

7. Urucum extract comprising between 2% and 92% by weight of a first molecule having formula (I) with R = CH₃ for its use in preventing the damage caused by exposure to ultraviolet rays on the skin of mammals, **characterised in that** it is administered orally.

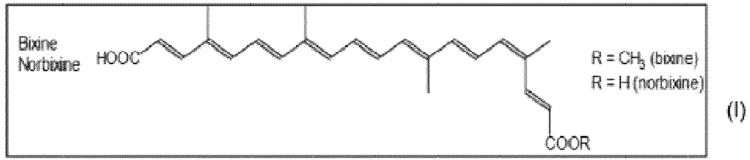

8. Urucum extract according to claim 7, said extract also comprising between 2% and 92% by weight of a second molecule having formula (I) with R = H.

9. Urucum extract comprising between 2% and 92% by weight of a first molecule having formula (I) with R = H for its use in preventing the damage caused by exposure to ultraviolet rays on the skin of mammals, **characterised in that** it is administered orally.

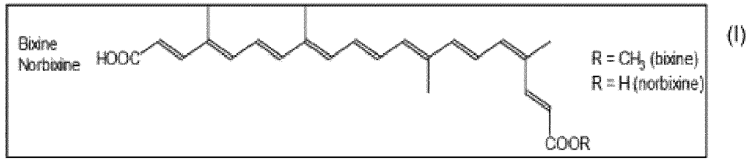

10. Urucum extract according to claim 9, said extract also comprising between 2% and 92% by weight of a second molecule having formula (I) with R = CH₃.

11. Urucum extract according to one of claims 7 to 8 or according to one of claims 9 to 10, said extract comprising at least 6% by weight of the first molecule.

12. Urucum extract according to claim 8 or according to claim 10, said extract comprising at least 6% by weight of the second molecule.
